# FASCICULE DE BREVET EUROPEEN

(11) **EP 0 447 303 B1**
(45) Date de publication et mention de la délivrance du brevet: **04.09.1996**
(21) Numéro de dépôt: 91400634.1
(22) Date de dépôt: 07.03.1991
(51) Int. Cl.: C12N 15/86, C07K 14/00

(54) **Virus herpès recombinants notamment pour la réalisation de vaccins, leur procédé de préparation, les plasmides réalisés au cours de ce procédé et les vaccins obtenus**
Rekombinante Herpes-Viren, besonders zur Herstellung von Impfstoffen, Verfahren zur Herstellung davon, während des Prozesses hergestellte Plasmide und durchgesetzte Impfstoffe
Recombinant herpes viruses, particularly for realization of vaccines, preparation process therefor, realized plasmids during this process and obtained vaccines

(30) Priorité: 12.03.1990 FR 9003105
(43) Date de publication de la demande: 18.09.1991
(73) Titulaire: RHONE MERIEUX S.A., 69002 Lyon (FR)
(72) Inventeur: Rey-Senelonge, Arielle, F-69140 Rillieux La Pape (FR); Kohen, Gilla, F-69140 Rillieux La Pape (FR)
(74) Mandataire: Bernasconi, Jean

(56) Documents cités:
- WO-A-88/07088
- J. of Virology, vol. 57, no. 3, mars 1986, pp. 802-808; M. Swain et D.A. Galloway
- J. of General Virology, vol. 71, no. 12, déc. 1990, pp. 2931-2939; F.J. Rixon et J. McLauchlan

## Description

Virus HVT recombinants notamment pour la réalisation de vaccins, leur procédé de préparation, les plasmides réalisés au cours de ce procédé et les vaccins obtenus.

La présente invention concerne des virus HVT recombinants pouvant être notamment utilisés dans des vaccins contre des maladies à virus de l'homme et des animaux, leur procédé de préparation, les plasmides réalisés au cours de ce procédé ainsi que les vaccins obtenus. Elle concerne encore une séquence nucléotidique correspondant à une partie du génome du virus herpès de la dinde (HVT) qui est susceptible d'être utilisée pour la préparation de tels virus.

Le virus herpès de la dinde (HVT) de la sous-famille des gamma-herpèsviridae est un virus naturellement apathogène et non-oncogène, relié sérologiquement au virus oncogène de la maladie de Marek, agent responsable d'une maladie lymphoproliférative des volailles, d'une importance économique considérable.

Ces deux virus possèdent de nombreuses homologies de séquence tout au long de leur génome et les données récentes publiées montrent, en outre, une similarité avec les génomes des virus herpès simplex (HSV) ou varicelle (VZV), ce qui suggère actuellement leur classification dans la famille des alpha-herpèsvirus, plutôt que celle des gamma-herpèsvirus, où ils étaient classés en raison de leur tropisme (1) (Pour les référence bibliographiques, voir annexe 1).

Pendant de nombreuses années, la vaccination à l'aide du virus HVT a été très efficace pour contrôler la maladie de Marek. Néanmoins, l'émergence de nouvelles souches hautement virulentes montre la nécessité d'utiliser des vaccins plus proches antigéniquement du virus sauvage. Dans ce contexte, les vaccins obtenus par manipulation génétique sont une possibilité intéressante.

De plus en plus se développent des vecteurs viraux vivants, tels que des souches atténuées de poxvirus ou d'herpèsvirus. Ainsi, les virus HSV, ou de la maladie d'Aujeszky (PRV) ont été utilisés comme vecteurs pour l'expression de gènes étrangers (26,34). Pour cela, les gènes étrangers ont été insérés dans des fragments clonés de régions non essentielles des génomes herpès, puis introduits dans le vecteur viral par recombinaison homologue. Cette dernière étape est effectuée simplement par cotransfection, puisque les ADNs des virus herpès sont naturellement infectieux.

Le virus HVT est un canditat de choix pour le développement d'un tel vecteur viral dans le domaine aviaire, puisqu'il présente le double avantage de pouvoir être utilisé pour ses propriétés vaccinales et comme vaccin d'autres maladies. De plus, ce virus entraîne une virémie permanente et peut être utilisé en embryo-vaccination.

Il est possible d'insérer à l'intérieur du génome HVT des gènes codant pour des immunogènes du virus de la maladie de Marek, potentialisant ainsi le rôle protecteur du virus HVT. Il est possible également d'insérer à l'intérieur du génome HVT des gènes codant pour des immunogènes d'agents pathogènes viraux, bactériens ou parasitaires d'autres maladies aviaires telles que la maladie de Marek (MDV), la bronchite infectieuse (IBV), la maladie de Newcastle (NDV), la peste aviaire, la maladie de Gumboro (IBDV), l'anémie aviaire (CAA), le syndrome chute de ponte, la coccidiose, la variole aviaire, la rhinotrachéite infectieuse, la colibacillose, la pasteurellose et l'hémophilose. Le virus HVT se présenterait alors comme une chimère multivalente.

Le matériel génétique des herpèsvirus est constitué par un ADN bicaténaire contenant 100 000 à 180 000 paires de bases. Différentes régions de ce génome ont été montrées comme étant non essentielles à la réplication virale, et sont donc des sites potentiels d'insertion de gènes étrangers, ou de délétion pour la création de nouvelles souches plus atténuées.

Certaines de ces régions sont associées à la virulence et leur modification provoque une diminution de la pathogénicité des virus. Ainsi, l'inactivation de la thymidine kinase rend l'herpès simplex humain non pathogène et n'empêche pas la croissance virale in vitro (3, 8, 21, 33) ; il en va de même pour le virus PRV (30). De plus, il a été montré que l'atténuation de la souche Bartha du virus PRV est liée à une délétion de la glycoprotéine gI dont le gène se trouve dans le petit fragment Us (18).

D'autres gènes des virus herpès ont été identifiés comme non essentiels à la croissance virale, sans que pour autant ils soient associés à des phénomènes de virulence. Parmi ces gènes, on peut citer le gène UL24 du virus HSV (23), différents gènes du virus HSV situés dans le petit fragment Us (35), le gène de la glycoprotéine gIII du virus (BHV) de la rhinotrachéite infectieuse bovine (9), le gène de la glycoprotéine gX du virus PRV (34).

La ribonucléotide réductase est une enzyme importante de la chaîne de biosynthèse de l'ADN, responsable de la réduction des ribonucléotides en désoxyribonucléotides. Les herpèsvirus possèdent une activité ribonucléotide réductase propre, répondant à des critères de régulation différents de ceux de l'enzyme cellulaire (12, 13). Tout comme l'enzyme bactérienne ou de mammifère, la ribonucléotide réductase des virus herpès consiste en 2 sous-unités hétérologues dont l'interaction est nécessaire à l'activité enzimatique : la grande sous-unité RR1 et la petite sous-unité RR2.

Les gènes codant pour ces protéines ont été localisés et séquencés pour le virus herpès simplex (HSV), le virus de la varicelle (VZV), le virus d'Epstein-Barr (EBV) et leur mode de transcription, étudié dans le cas du virus HSV (15, 16, 27, 28). Récemment Goldstein et Weller (1988), en insérant le gène lacZ de la bêta-galactosidase en phase dans la région terminale du gène codant pour la grande sous-unité de la ribonucléotide réductase du virus HSV ont montré le caractère non essentiel de ce gène. Des mutants délétés dans ce même gène du virus HSV ont été construits et leur étude prouve que la ribonucléotide réductase n'est pas nécessaire à la multiplication virale dans des cellules en phase exponentielle de croissance, l'enzyme d'origine cellulaire pouvant alors compenser cette délétion (4, 5).

Néanmoins, même lorsqu'ils sont cultivés sur des cellules possédant une activité ribonucléotide réductase transcomplémentante, les mutants délétés voient leur croissance modifiée (5). Ce phénomène est considérablement amplifié lorsque ces mutants sont cultivés à 39,5°C ou sur des cellules privées de sérum (20). Plus récemment encore, il a été montré que la délétion de la grande sous-unité de la ribonucléotide réductase entraîne une diminution de la virulence du virus HSV chez la souris (7).

Il n'existe, à ce jour, aucune donnée publiée concernant le rôle essentiel de la ribonucléotide réductase du virus HVT. Aucune séquence n'existe concernant la petite sous-unité RR2 du gène de la ribonucléotide réductase du virus HVT.

Ainsi, la demande de brevet WO-A-88 07088 décrit l'introduction d'un gène d'intérêt dans une région non essentielle du virus HVT ou du virus MDV sérotypes 1 et 2. Cette demande propose de manière spécifique d'insérer dans le gène codant pour la thymidine-kinase (TK) ou dans celui codant pour la protéine A.

L'invention a donc pour objectif de fournir des virus recombinants ou virus chimères susceptibles de se multiplier de façon normale, notamment pour la réalisation de vaccins efficaces.

Le clonage et le séquençage de la partie du génome du virus herpès de la dinde (HVT) qui correspond au gène de la petite sous-unité RR2 de la ribonucléotide réductase ont d'abord été réalisés.

De façon surprenante, le gène codant pour la petite sous-unité RR2 a pu être muté ou totalement délété et remplacé par une séquence codante, sans que le mutant ainsi créé présente une altération en ce qui concerne sa croissance. Il a ainsi été démontré que le virus HVT peut être effectivement utilisé comme vecteur d'expression, en insérant un gêne étranger dans le gène de la petite sous-unité RR2 et cela notamment sous le contrôle du ou des promoteurs de la petite sous-unité RR2 de la ribonucléotide réductase.

L'invention a donc pour objet la séquence nucléotidique, et ses variantes, qui correspond au gène de la petite sous-unité RR2 de la ribonucléotide réductase du virus HVT.

Cette séquence peut être associée à d'autres fragments usuels tels que promoteurs, signaux d'initiation ou d'arrêts ou introns ou autres séquences non codantes à l'extrémité 3' et/ou 5'. Elle inclut également les variantes obtenues notamment par substitution de codons ou de nucléotides conservant la signification du code, ou par substitutions, insertions ou délétions codant pour un polypeptide équivalent et notamment conservant l'antigénicité du polypeptide. Elle inclut également tout fragment permettant l'expression d'un polypeptide conservant cette antigénicité.

L'invention concerne également les différents fragments de restriction ou synthétiques, provenant de la séquence selon l'invention et notamment tout fragment capable de s'hybrider sur le gène de la petite sous-unité RR2 de HVT ou tout autre virus herpès.

L'invention a également pour objet un virus recombinant HVT comprenant un gène hétérologue inséré dans la région du génome du virus correspondant au gène de la petite sous-unité RR2 de la ribonucléotide réductase, de façon à pouvoir être exprimé. Par gène hétérologue, on entend notamment un gène codant pour une protéine ou glycoprotéine immunogène d'un agent pathogène viral, bactérien ou parasitaire. Dans le cas d'un vecteur viral constitué du virus HVT, le gène hétérologue pourra être notamment un gène codant pour un immunogène du virus de la maladie de Marek, de la bronchite infectieuse aviaire, de la maladie de Newcastle, de la peste aviaire, de la maladie de Gumboro, de l'agent de la coccidiose, de la laryngotrachéite infectieuse, de la colibacillose.

Par gène hétérologue, on entend également tout autre gène étranger au virus HVT et codant pour un peptide ou protéine, par exemple, hormones, facteurs de croissance, etc.

Le gène hétérologue inséré est exprimé de préférence sous le contrôle des séquences régulatrices de la transcription du gène de la petite sous-unité RR2. On peut cependant faire en sorte que cette expression soit sous la dépendance de séquences promotrices, provenant du virus considéré ou d'autres virus herpès, rapportées dans le génome dudit virus considéré. Le gène est placé en aval des signaux d'initiation dans un cadre de lecture correct pour son expression.

De préférence, on substitue les codons d'initiation et de terminaison du gène RR2 par ceux du gène à insérer.

L'invention a encore pour objet un procédé de préparation d'un virus HVT recombinant, dans lequel on insère le gène hétérologue dans la région, du génome de HVT, qui correspond au gène de la petite sous-unité RR2 de la ribonucléotide réductase.

De préférence, on isole la partie du génome viral enfermant le gène de la petite sous-unité RR2, on réalise, de préférence, une délétion partielle ou totale de ce gène et l'on insère le gène hétérologue dans la région correspondant audit gène avant d'introduire le fragment ADN ainsi obtenu dans le génome du virus par cotransfection et recombinaison homologue.

De préférence, on conserve les signaux d'initiation et de terminaison de transcription du gène de la petite sous-unité RR2. Cette expression peut aussi se faire sous la dépendance de séquences promotrices, provenant du virus considéré, par exemple le promoteur du gène RR1, du gène TK, du gène gA, du gène gB, ou d'autres virus herpès, par exemple le promoteur du gène gI du virus BHV ou du gène gII du virus PRV, rapportées dans le génome du virus considéré.

Dans un mode de réalisation , un procédé de construction d'un virus recombinant peut comprendre les étapes suivantes ;
a) on isole un fragment Bam HI K1 du génome de HVT par digestion du génome par l'enzyme de restriction Bam HI,
b) on digère ce fragment par l'enzyme de restriction Hind III, pour obtenir un fragment correspondant à la partie 5' du gène RR2 et à la région en amont incluant le promoteur et un fragment correspondant à la partie 3' du gène RR2 et à la région en aval de ce gène,
c) on clone les deux fragments ainsi obtenus en b) respectivement dans les vecteurs pUC 18 et pUC 19,
d) on digère ces plasmides respectivement par les couples d'enzymes de restriction Hind III - Aat II et Xmn I - Aat II pour générer un nouveau plasmide comportant une délétion entre les sites Hind III et Xmn I,
e) on crée par mutagénèse dirigée des sites de restriction dans le plasmide obtenu en d), permettant l'insertion correcte du gène à exprimer,
f) on clone dans ces sites de restriction un gène hétérologue, notamment un gène codant pour un immunogène associé à une maladie aviaire, et
g) on insère le fragment d'ADN obtenu en f) dans le génome du virus HVT par cotransfection et recombinaison homologue.

L'invention a encore pour objet un virus recombinant obtenu par le procédé selon l'invention.

L'invention a encore pour objet un plasmide comportant une partie du génome, du virus HVT, qui renferme le gène de la petite sous-unité RR2.

De préférence, la partie de génome contenu dans le plasmide comprend une délétion dans la région correspondant au gène de la petite sous-unité RR2. De préférence encore, un gène hétérologue, par exemple un gène codant pour un immunogène, est inséré dans ladite région correspondant au gène de la petite sous-unité RR2.

L'invention a encore pour objet un plasmide comportant le fragment Bam HI K1, du virus HVT, renfermant le gène de la petite sous-unité RR2 de la ribonucléotide réductase. De préférence, ce fragment comprend une délétion de 766 bases entre les sites initiaux Hind III et Xmn I. De préférence encore un gène hétérologue codant pour un immunogène de maladie aviaire est inséré dans la région, de ce fragment, qui correspond au gène de la petite sous-unité RR2 de la ribonucléotide réductase.

L'invention a encore pour objet des virus recombinants HVT comprenant le fragment de génome recombiné du plasmide réalisé à partir du génome dudit virus.

L'invention a encore pour objet un vaccin comprenant un virus recombinant obtenu comme précédemment. Les excipients et diluants seront notamment ceux utilisés habituellement pour la préparation de tels vaccins, notamment des vaccins vivants concernés.

L'invention va être maintenant décrite plus en détail. On va d'abord décrire le clonage et le séquençage du gène de la petite sous-unité RR2 de la ribonucléotide réductase du virus HVT, puis l'introduction d'une délétion dans le gène de la petite sous-unité de HVT et la démonstration du caractère non essentiel de cette petite sous-unité dans la multiplication virale. On verra ensuite l'insertion de gènes hétérologues dans le vecteur viral et leur expression.

La description détaillée sera faite en reférence au dessin annexé dans lequel :

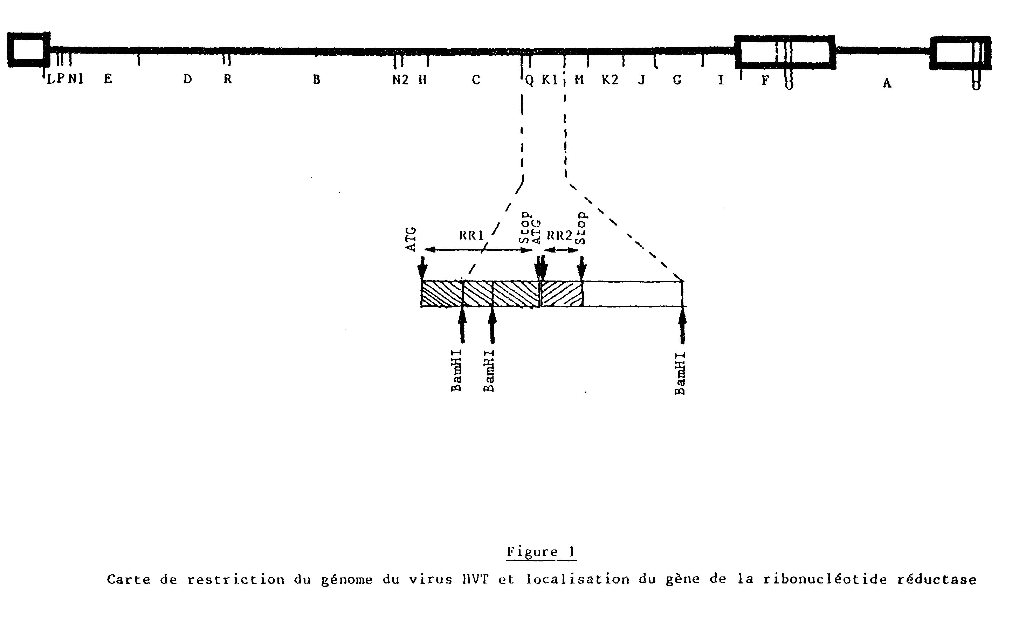

la figure 1 représente la carte de restriction du génome du virus HVT et la localisation du gène RR2 ;
la figure 2 représente un schéma expliquant l'introduction d'une délétion dans la petite sous-unité RR2 de la ribonucléotide réductase ;
la figure 3 représente un schéma expliquant la création de sites de clonage par mutagénèse dirigée dans le gène délété de la petite sous-unité RR2 ;
la figure 4 représente un schéma expliquant l'insertion du gène LacZ à la place du gène codant pour la petite sous-unité RR2 de la ribonucléotide réductase ;
la figure 5 représente un schéma expliquant l'introduction d'un "polylinker" à la place du gène RR2 ; et

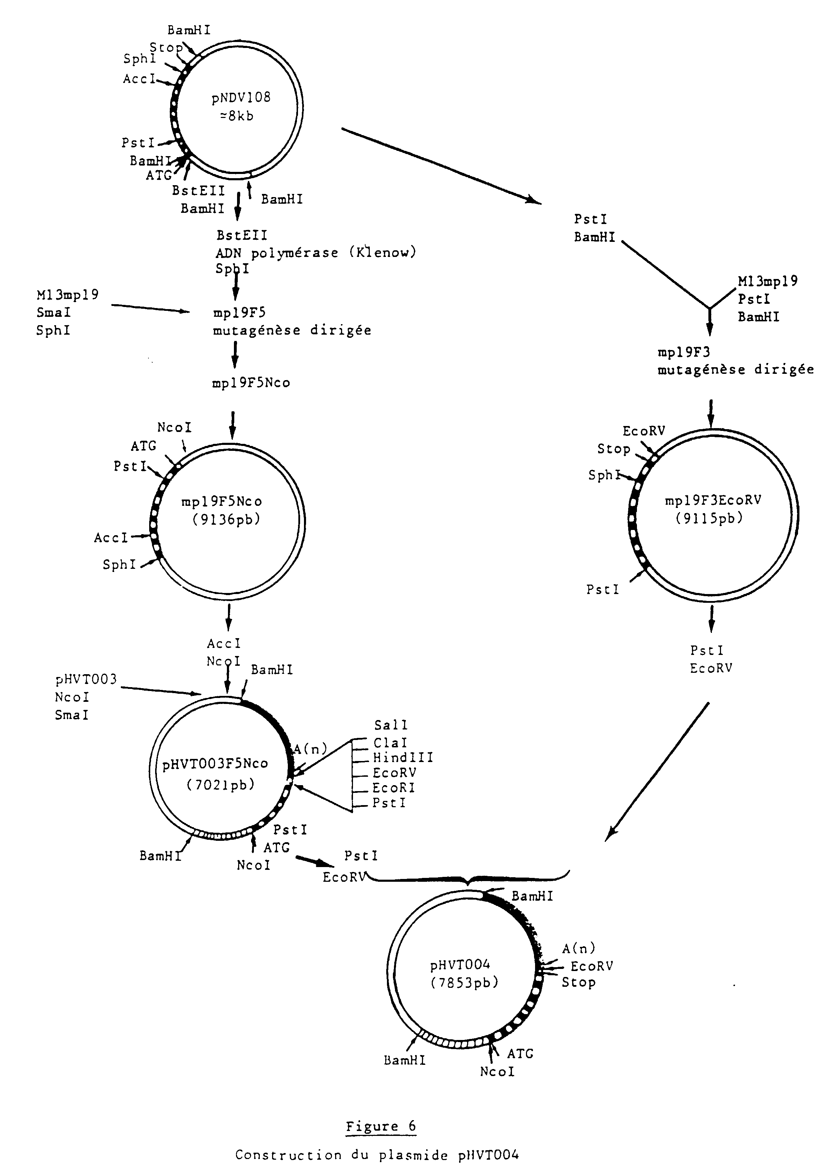

la figure 6 représente un schéma expliquant l'insertion de l'ADNc du gène de la protéine fusion du virus de la maladie de Newcastle.

### MATERIEL ET METHODES

De manière générale, les techniques utilisées pour la construction des plasmides recombinants sont celles décrites par T. Maniatis et al. (17). Pour toutes les étapes de clonage ou de sous-clonage, le vecteur linéarisé par les enzymes de restriction appropriées est déphosphorylé avant ligation.

La purification des fragments d'ADN à partir d'un gel d'agarose est faite selon la technique décrite par le fabricant : "Geneclean" (Bio 101, San Diego, Californie, USA).

### Souche virale

La souche FC 126 du virus HVT a été isolée en 1968 par le Dr. Witter dû Regional Poultry Research Laboratory (USDA, East Lansing, Michigan, USA), dans un troupeau de dindes de 23 semaines (36).

Elle a été ensuite passée 10 fois sur des fibroblastes de canard, puis a subi 9 passages supplémentaires sur des fibroblastes d'embryons de poulets EOPS.

L'ADN viral utilisé pour ce travail a été extrait à partir de virus ayant fait l'objet au total de 23 à 24 passages à partir de l'isolat d'origine.

### Culture cellulaire

Les fibroblastes d'embryons de poulets sont cultivés en milieu F10-199 (Rhône-Mérieux, Lyon, France) supplémenté de sérum de veau foetal.

Les cellules destinées à produire le virus utilisé pour purifier l'ADN ont été cultivées en flacons roulants.

Pour les expériences de transfection, les cellules ont été cultivées en boîtes de Pétri de 10 cm ou sur des boîtes à 24 puits.

### Isolement d'ADN du virus HVT pour le clonage

Les monocouches confluentes de fibroblastes de poulet sont infectées par le virus HVT et laissées en incubation pendant 1 à 3 jours à 39°C + 1°C. Lorsque l'effet cytopathique dû au virus est jugé optimum, les cellules infectées sont décollées des parois des flacons roulants puis centrifugées. Les culots de cellules infectées sont remis en suspension dans un stabilisateur contenant du saccharose et de l'albumine bovine. La suspension cellulaire ainsi préparée est plongée dans un bain-marie glacé, puis traitée aux ultrasons. La suspension est ensuite clarifiée par centrifugation à basse vitesse et centrifugée 1 h à 40 000 tr/mn avec un rotor Ti 45 (Beckmann, Palo Alto, Californie, USA).

Le culot viral, repris en tampon (Tris 10 mM; NaCl 136 mM ; KCl 2,6 mM ; MgCl₂ 20 mM; CaCl₂ 1,8 mM) et purifié par centrifugation sur gradient zonal de saccharose (30 %, 50 % p/v) pendant 20 h à 23 000 tr/mn avec un rotor SW28 (Beckmann). Le virus obtenu dans la fraction correspondant à une densité de saccharose correspondant à une concentration d'environ 48 % est récupéré. Après dilution en tampon Tris 50 mM EDTA 10 mM, le virus est sédimenté par centrifugation pendant 40 mn à 40 000 tr/mn avec un rotor SW41 (Beckmann). L'ADN viral est alors extrait en traitant le virus purifié par la protéinase K à raison de 100 µg/ml pendant 2 h à 37° C en présence de SDS à 0,5 %, puis purifié par un traitement au phénol suivi de 3 traitements au chloroforme-alcool isoamylique (24:1). L'ADN viral est ensuite précipité par de l'éthanol à -20°C.

### Isolement d'ADN infectieux pour les expériences de transfection.

Le virus HVT associé aux cellules est inoculé à des monocouches confluentes de fibroblastes de poulets à une multiplicité d'infection d'environ 0,001 ufp (unité formatrice de plaques) par cellule.

Les cellules infectées sont incubées à 37°C en milieu F10-199 supplémenté avec 2 % de sérum de veau foetal.

Lorsque l'effet cytopathique est maximum (2 à 3 jours), le milieu est éliminé et les cellules infectées sont récoltées après trypsination, puis sédimentées par centrifugation à basse vitesse.

Le culot renfermant les cellules infectées est repris en tampon Tris 10 mM - EDTA 1 mM, pH 8, renfermant 0,5 % (p,v) de Triton X100 et 0,5 % (p,v) de NP40 à raison de 2.10⁸ cellules infectées par ml de tampon et traitées comme décrit par Lee et coll, 1980 (14).

Le virus est ensuite obtenu par centrifugation sur gradient de saccharose 15 % - 30 % (p/v) pendant 24 mn à 22 000 tr/mn comme indiqué par Lee et coll.

L'ADN viral est extrait des virus purifiés par traitement à la protéinase K (400 ug/ml final) et SDS 0,5 % (p,v) pendant 1 nuit à 37°C, puis purifié par centrifugation zonale sur gradient de glycérol 10 % - 30 % (v,v), pendant 4 h 30 à 38 000 tr/mn avec un rotor SW41.

L'ADN viral est ensuite précipité par l'alcool et repris en tampon Tris 10 mM, EDTA 1 mM, pH 7,5.

### Clonage de l'ADN

L'ADN viral a été digéré par l'enzyme de restriction Bam HI et les fragments ont été clonés dans le plasmide pUC 18 (38) digéré préalablement par Bam HI et déphosphorylé.

Les bactéries E. coli NM 522 (6) ont été transformées selon la procédure au chlorure de calcium, puis cultivées en présence d'ampicilline et d'Xgal.

Les colonies blanches ont été cultivées en petits volumes et l'ADN plasmidique a été extrait.

Les clones ont été sélectionnés en fonction de la taille des inserts déterminée par électrophorèse sur gel d'agarose à 0,8 % après digestion par Bam HI.

### Séquençage.

Les séquences nucléotidiques ont été déterminées selon la technique des didésoxynucléotides décrite par Sanger, 1977 (24), à l'aide du kit Sequanase version 2 (USB, Cleveland, Ohio, USA) selon le protocole décrit par le fabricant (29), en utilisant des oligonucléotides synthétiques spécifiques (Applied Biosystems, Forster City, Californie, USA).

### Mutagénèse dirigée.

- Les fragments d'ADN clonés dans le phage M13 ont été mutagénisés selon la technique décrite par le fabricant du kit de mutagénèse in vitro (Amersham, Buckinghamshire, Grande-Bretagne) (19, 31, 32).
- Les fragments d'ADN clonés dans le vecteur Blue Script SK+ (Stratagene, La Jolla, Californie, USA) ont été mutagénisés après séparation des ADN simple brin à l'aide du phage helper R408 (Stratagene, La Jolla, USA) (22). La procédure de mutagénèse et de sélection des mutants en utilisant la souche CJ236 dut⁻ ung⁻ d'E. coli (In Vitrogen, San Diego, Californie, USA), a été décrite par T. Kunkel et al. (10,11).

### Transfection.

Les cellules primaires d'embryons de poulets cultivées jusqu'à confluence (24 h) ont été cotransfectées par l'ADN viral HVT et l'ADN d'un plasmide renfermant le gène à insérer, flanqué en 5′ et 3′ de régions du génome HVT de façon à permettre la recombinaison homologue.

La technique utilisée est celle de la Lipofectine® décrite par le fabricant (BRL, Bethesda Research Laboratory, Gaithersburg, Madison, USA) (2).

1 µg d'ADN viral a été mélangé avec 1 à 10 µg d'ADN du plasmide linéarisé, sous un volume de 50 µl, puis ajouté à 50 µl de réactif Lipofectine.

L'ensemble a été laissé 30 mn à température ambiante, puis ajouté aux cellules préalablement rincées par du milieu sans sérum. L'adsorption a été réalisée pendant 5 h à 37°C sous ambiance CO₂ en présence de 3 ml de milieu sans sérum (optimum de BRL). Après 5 h, du sérum de veau foetal a été ajouté à 8 % final et la culture a été poursuivie pendant 72 h ou plus.

Les premières figures d'effet cytopathogène sont apparues au bout de 72 h.

Les cellules ont parfois été trypsinées après 72 h, puis réinoculées (1:1 ou 1:2) en un passage secondaire, en milieu F10-199, et incubées à 37°C jusqu'à apparition des plages de lyse (72 h au minimum), cela pour augmenter l'effet cytopathogène.

200 plages en moyenne par µg d'ADN HVT ont été obtenues.

Après apparition des foyers viraux, le milieu est éliminé puis remplacé par du milieu F10-199 supplémenté par 2 % de sérum de veau foetal et renfermant 1 % d'agarose.

Les cellules infectées ont été récupérées par carottage, dissociées par addition d'une goutte de solution de trypsine (0,4 ‰) et inoculées à une culture cellulaire.

### EXEMPLE 1.

### CLONAGE DU GENE DE LA RIBONUCLEOTIDE REDUCTASE ET DETERMINATION DE SA SEQUENCE NUCLEOTIDIQUE

Il a été montré par Buckmaster et coll. que le gène de la grande sous-unité de la ribonucléotide réductase du virus HVT pouvait être localisée soit au niveau du fragment de restriction Bam HI K1, soit au niveau du fragment Bam HI K2 (1).

Les plasmides pUC 18 renfermant un insert d'une taille correspondant à celle du fragment K1/K2 (soit 4,2 kilobases) décrits ci-dessus (clonage de l'ADN), ont donc été sélectionnés, puis les extrémités des inserts séquencées. Les séquences obtenues ont été comparées, à l'aide d'un programme d'analyse de sequences Microgenie (Beckmann), aux séquences publiées de la ribonucléotide réductase des virus VZV, HSV, EBV. L'homologie de séquence a permis de retenir le plasmide pHVT K1. Ce plasmide contient la totalité du gène de la petite sous-unité RR2 de la ribonucléotide réductase et la régon 3′ du gène de la grande sous-unité RR1.

La séquence du gène de la ribonucléotide réductase est repérée SEQ ID NO : 1 dans la liste des séquences annexée (annexe 2). La phase de lecture correspondant au gène de la petite sous-unité RR2 commence à la position 1965 et se termine à la position 2759.

La séquence RR2 obtenue présente une homologie de 54 % au niveau nucléotides avec la séquence correspondante du virus VZV. Cette homolopie n'est plus que de 30 % avec le virus EBV, ce qui montre à nouveau une plus grande homologie des séquences du virus HVT avec celles des alpha-herpèsvirus.

### EXEMPLE 2.

### INSERTION DU GENE Lac Z DANS LE VECTEUR HVT

La possibilité d'utiliser le gène de la petite sous-unité de la ribonucléotide réductase comme site d'insertion de gènes étrangers peut être démontrée en lui substituant tout d'abord un gène marqueur, par exemple le gène lacZ de la bêta-galactosidase.

Si le site est bien non essentiel, le virus mutant HVT Lac RR2⊖ après recombinaison in vivo donnera en présence d'Xgal une coloration bleue.

### 1 - Construction d'un plasmide pHVT002 renfermant le gène lacZ à la place du gène de la petite sous-unité de la ribonucléotide réductase.

Ce plasmide a été construit en 3 étapes :
- introduction d'une délétion dans le gène de la petite sous-unité de la ribonucléotide réductase : plasmide pHVT 001 ;
- création de sites de restriction par mutagénèse dirigée pour le clonage du gène hétérologue ;
- insertion du gène lacZ de la bêta-galactosidase : plasmide pHVT 002.

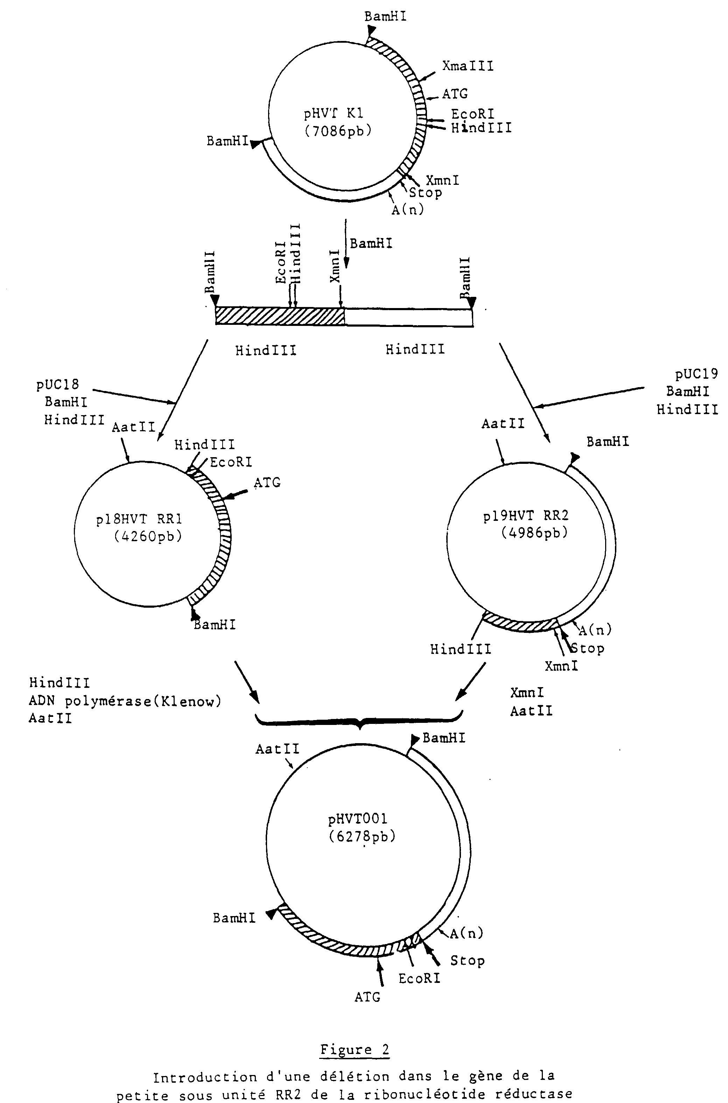

### 1.1 - Construction du plasmide pHVT001 (figure 2).

Le plasmide pHVT K1, qui renferme le fragment de restriction BamHI K1, a été digéré par BamHI. Ce fragment de 4,2 kilobases a été purifié par électrophorèse sur gel d'agarose. Il a ensuite été digéré par HindIII.

Le fragment BamHI-HindIII de 1374 paires de bases a été sous-cloné dans le vecteur pUC18 préalablement linéarisé pour donner le plasmide p18HVT RR1.

Le fragment HindIII-BamHI de 2826 paires de bases a été sous-cloné dans le vecteur pUC19 (38) préalablement linéarisé pour donner le plasmide p19HVT RR2.

Le plasmide p18HVT RR1 a été digéré par HindIII. Les extrémités de l'ADN ainsi linéarisé ont été remplies grâce à l'ADN polymérase (fragment Klenow). Une deuxième digestion par l'enzyme AatII a ensuite été réalisée. Le fragment de 3578 paires de bases ayant une extrémité franche et l'autre extrémité correspondant au site AatII a été purifié par électrophorèse sur gel d'agarose.

Le plasmide p19HVT RR2 a été digéré par l'enzyme de restriction XmnI, puis par l'enzyme de restriction AatII. Le fragment de restriction de 2700 paires de bases AatII-XmnI, après purification sur gel d'agarose, a été ligué avec le fragment de 3578 paires de bases décrit au paragraphe précédent pour donner le plasmide pHVT001. Ce plasmide comporte une délétion de 766 paires de bases entre les sites initiaux HindIII et XmnI.

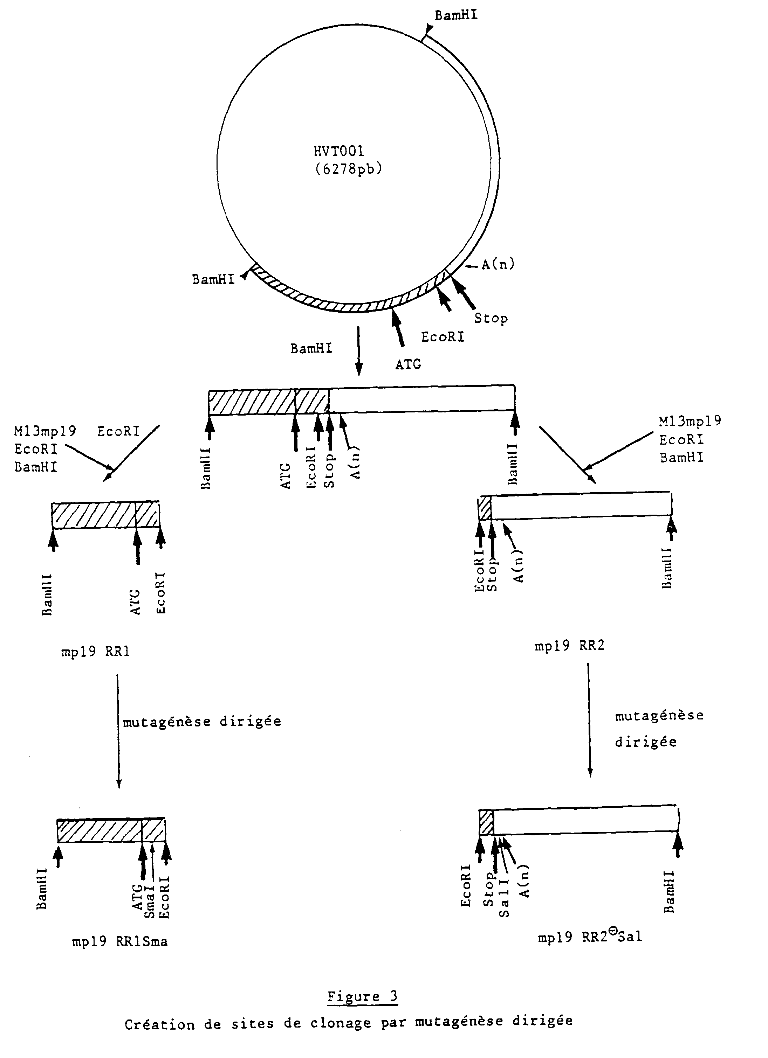

### 1.2 Création de sites de clonage par mutagénèse dirigée (figure 3)

Le fragment de restriction BamHI de 3440 paires de bases issu du plasmide pHVT001 a été purifié par électrophorèse, puis digéré par EcoRI. Les 2 fragments BamHI- EcoRI de respectivement 1340 paires de bases et 2100 paires de bases environ ont été clonés dans le phage M13mp19 (38). Les clones obtenus sont respectivement nommés mp19 RR1 et mp19 RR2⊖.

Un site SmaI a été créé par mutagénèse dirigée dans le phage mp19 RR1, en 3′ de l'ATG, à l'aide de l'oligonucléotide désigné par SEQ ID NO : 3 dans la liste des séquences annexée.

Le phage ainsi obtenu est dénommé : mp19 RR1 Sma.

Un site SalI a été introduit dans le phage mp19 RR2⊖ en 5′ du signal de polyadénylation du gène de la petite sous-unité à l'aide de l'oligonucléotide désigné par SEQ ID NO : 4.

Le phage ainsi obtenu est dénommé mp19 RR2⊖ Sal.

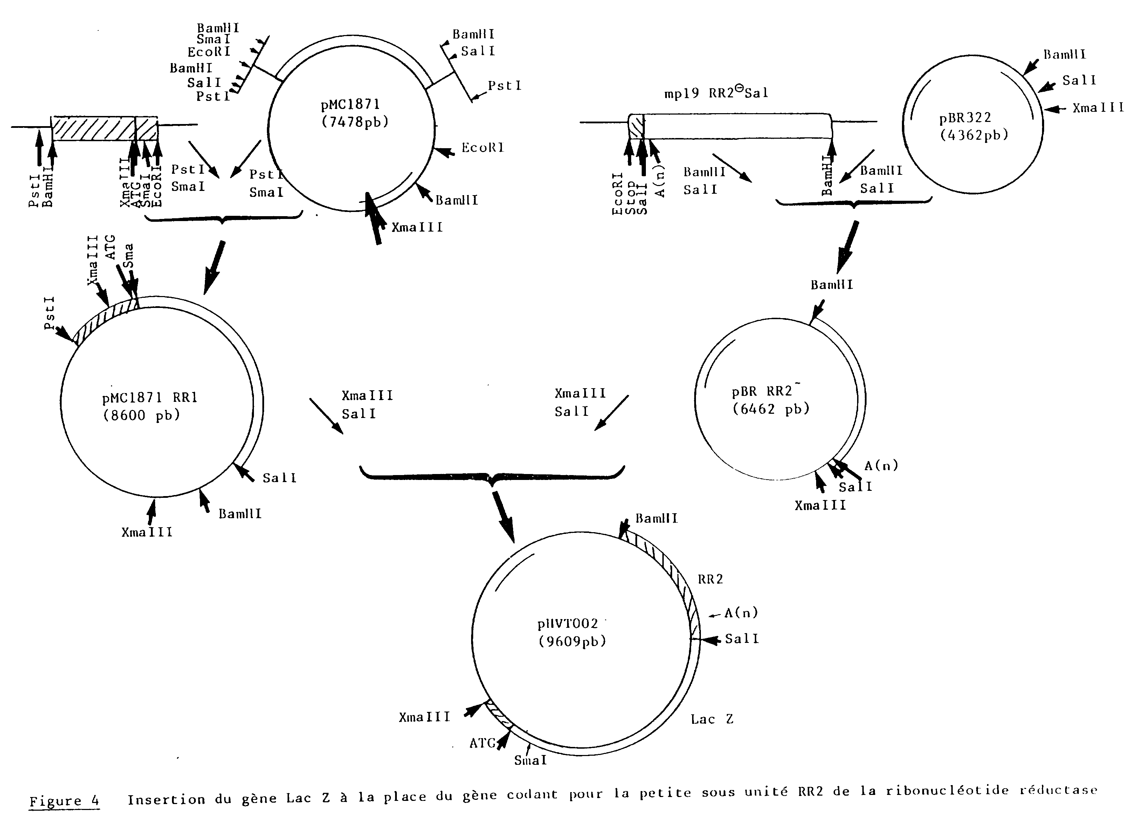

### 1.3 Création du plasmide pHVT002 (figure 4).

Le fragment muté BamHI-EcoRI de 1340 paires de bases contenu dans le vecteur mp19 RR1Sma, tel que décrit à la figure 3, a été purifié par électrophorèse après digestion par PstI et SmaI. Il a ensuite été cloné dans le vecteur pMC1871 (Pharmacia-LKB, Uppsala, Suède) (25) entre les sites PstI et SmaI, pour donner le plasmide pMC1871 RR1.

Ce plasmide renferme les 1000 bases 3′ terminales du gène de la grande sous-unité RR1 de la ribonucléotide réductase et le gène Lac Z en phase avec l'ATG de la petite sous-unité RR2.

Le fragment muté BamHI-EcoRI d'environ 2100 paires de bases provenant du phage mp19 RR2⊖ Sal tel que décrit à la figure 3, a été cloné dans le vecteur pBR322 (37) entre les sites BamHI et SalI pour donner le plasmide pBR RR2⊖. Les deux nouveaux plasmides pMC1871 RR1 et pBR RR2⊖ ont été digérés par XmaIII et SalI.

Le fragment XmaIII-SalI de 3665 paires de bases isolé à partir du plasmide pMC1871 RR1 a été purifié par électrophorèse. De la même façon, le fragment XmaIII-SalI de 6200 paires de bases environ isolé à partir du plasmide pBR RR2⊖ a été purifié. Ces deux fragments ont ensuite été ligués pour donner le plasmide pHVT002.

Ce plasmide renferme donc les 200 paires de bases de la partie 3′ du gène RR1, les 33 paires de bases non codantes jusqu'à l'ATG du gène RR2, puis le gène Lac Z inséré en phase, et enfin la région 3′ non codante du gène RR2.

### 2. Obtention d'un virus HVT muté RR2⊖ Lac Z

Des cellules primaires d'embryons de poulet ont été cotransfectées avec 1 à 10 µg de plasmide pHVT002 et 1 ug d'ADN génomique HVT infectieux selon la technique de la Lipofectine. Après 72 à 96 h de culture à 37°C sous ambiance CO₂, de nombreuses figures d'effet cytopathique sont observées. Le milieu a alors été éliminé et remplacé par une surcouche d'agarose à 1 % en milieu F10-199 renfermant 0,5 % d'Xgal. La culture a été poursuivie pendant 24 h. Au bout de 4 à 5 h, des plages bleues nettement identifiables ont été obtenues.

Les cellules ont alors été prélevées par carottage et le tapis cellulaire restant au fond du puits a été repris par addition d'une goutte de trypsine. L'ensemble a été inoculé à des cellules saines. Un effet cytopathique a été observé après 72 à 96 h. APrès addition d'Xgal, les cellules infectées présentent des lésions du tapis cellulaire colorées en bleu.

### EXEMPLE 3

### INSERTION DU GENE FUSION DU VIRUS DE LA MALADIE DE NEWCASTLE

L'insertion du gène fusion de la maladie de Newcastle a été réalisée en deux étapes.
1) Construction du plasmide pHVT003 comprenant la délétion du gène RR2 et l'insertion de plusieurs sites de clonage.
2) Construction du plasmide pHVT004 contenant le gène fusion à la place du gène RR2.

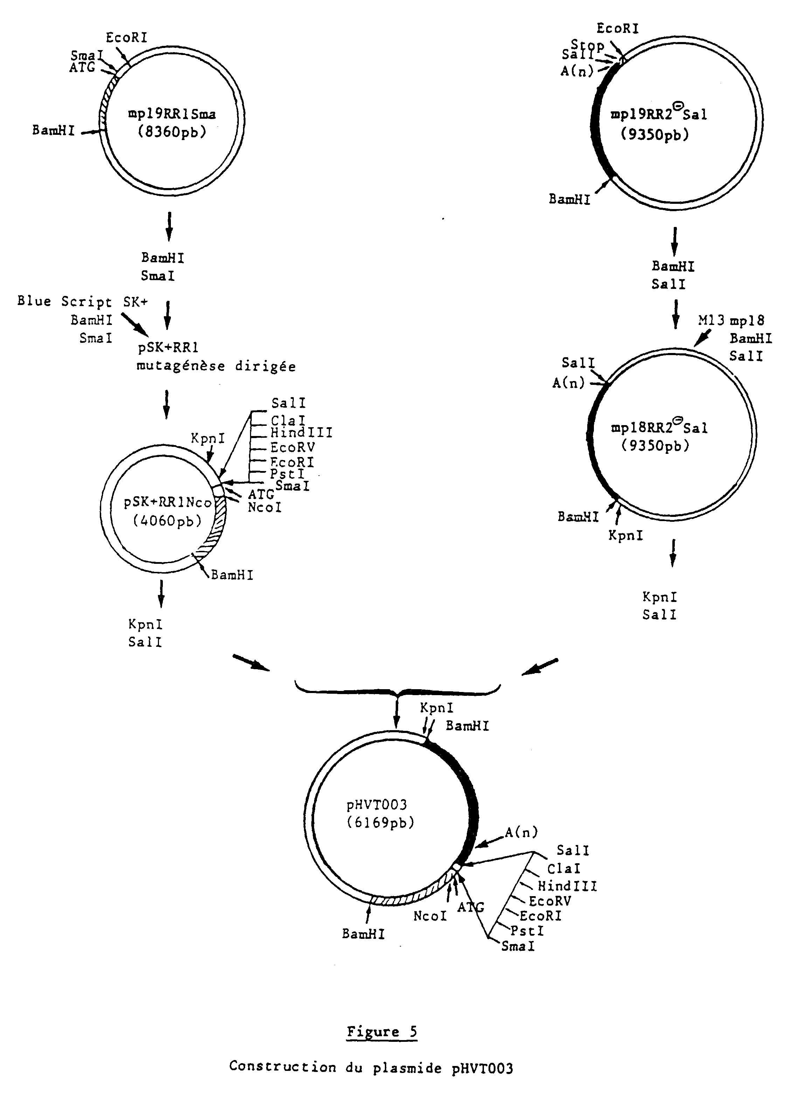

### 1. Construction du plasmide pHVT003 (figure 5)

Le phage recombinant muté mp19 RR1Sma décrit à la figure 3 a été digéré par SmaI et BamMHI

Le fragment libéré de 1110 paires de bases, qui contient une partie du gène RR1 et le codon ATG du gène RR2, a été cloné dans le vecteur Blue Script SK+ au niveau des sites de restriction SmaI et BamHI pour donner le plasmide pSK+RR1. Par mutagénèse dirigée, un site de restriction NcoI a été introduit au niveau du signal ATG à l'aide de l'oligonucléotide SEQ ID NO : 5, générant ainsi le plasmide pSK+ RR1Nco.

Le phage recombinant muté, mp19 RR2⊖Sal, décrit figure 3, a été digéré par BamHI et SalI. Le fragment libéré de 2,1 kilobases environ a été cloné dans le vecteur M13mp18 au niveau des sites de restriction BamHI et SalI. Le clone obtenu a été appelé mp18 RR2-Sal.

Le fragment de 2,1 kilobases environ a été libéré du phage mp18 RR2-Sal par digestion par KpnI et SalI, puis introduit au niveau de ces sites dans le plasmide pSK+ RR1Nco, linéarisé par les enzymes KpnI et SalI. Un plasmide pHVT003 a ainsi été construit. Il renferme une partie du gène RR1, la région 3′ non codante de ce gène, le codon ATG du gène RR2, une séquence comprenant plusieurs sites de restriction uniques et enfin la région 3′ non codante du gène RR2.

### 2. Construction du plasmide pHVT004 qui renferme le gène fusion du virus de la maladie de Newcastle, inséré à la place du gène RR2.

L'ADNc du gène fusion a été cloné dans le vecteur pBR322 au site ScaI, ce qui correspond au plasmide pNDV108. La séquence du gène fusion est donnée en annexe : SEQ ID NO : 2

Le plasmide pNDV108 a été digéré par BstEII, les extrémités remplies par l'ADN polymérase (fragment Klenow) puis par SphI. L'insert de 1786 paires de bases ainsi libéré a été cloné dans le phage M13mp19 linéarisé par SmaI et SphI pour donner le phage mp19 F5. Ce phage est ensuite mutagénisé à l'aide de l'oligonucléotide SEQ ID NO : 6, pour donner le phage muté mp19 F5Nco.

Le phage mp19 F5Nco a été digéré par AccI et NcoI, et le fragment de 852 paires de bases ainsi obtenu a été cloné dans le vecteur pHVT003 linéarisé par NcoI et SmaI pour donner le plasmide pHVT003 F5Nco.

Ce plasmide insère donc la région 3′ terminale du gène RR1, les 33 bases non codantes en 3′ de ce gène, puis 852 bases du gène fusion inséré en phase, une séquence "polylinker" et enfin la région non codante située en 3′ du gène RR2.

Le plasmide pNDV108 a été digéré par PstI et BamHI. Le fragment de 1765 paires de bases obtenu a été cloné dans le phage M13mp19 linéarisé par PstI et BamHI pour donner la phage mp19 F3, puis mutagénisé à l'aide de l'oligonucléotide SEQ ID NO : 7, pour donner le phage mp19 F5EcoRV. Ce phage a été digéré par PstI et EcoRV, et le fragment de 1567 paires de bases ainsi libéré à été cloné dans le plasmide pHVT003 F5Nco, linéarisé par PstI et EcoRV, pour donner le plasmide pHVT004.

Ce plasmide contient la région 3' du gène RR1, les 33 bases non codantes en 3' de ce gène, puis le gène fusion du virus de la maladie de Newcastle inséré en phase, et enfin la région 3' non codante du gène RR2.

### INSERTION DU GENE FUSION DANS LE VIRUS VECTEUR HVT

Le plasmide pHVT004 a été utilisé dans des expériences de cotransfection de cellules de fibroblastes d'embryons de poulet avec l'ADN infectieux du virus HVT. On peut ainsi obtenir un virus recombinant HVT exprimant le gène fusion du virus de la maladie de Newcastle.

La petite sous-unité de la ribonucléotide réductase peut donc être utilisée pour insérer des gènes dans le génome du virus HVT et les promoteurs de la ribonucléotide réductase sont efficaces pour permettre leur expression.

On peut ainsi insérer des gènes codant pour des immunogènes associés à la maladie de Marek, à la bronchite infectieuse aviaire, à la maladie de Newcastle, à la peste aviaire, à la maladie de Gumboro, à la coccidiose, à la rhinotrachéite infectieuse, à la colibacillose.

Les virus recombinants ainsi obtenus pourront être utilisés comme vecteurs d'expression du gêne inséré, dans des vaccins dits vaccins chimères. Le virémie permanente dont est responsable le virus HVT doit permettre une bonne dissémination des immunogènes exprimés et ainsi provoquer la réponse immunitaire souhaitée.

Bien entendu, les virus recombinants selon l'invention peuvent comporter ensuite, en plus du ou des gènes insérés dans le gène de la petite sous-unité RR2, un ou plusieurs gènes insérés en d'autres endroits du génome, par exemple dans la grande sous-unité RR1 ou dans le gène codant pour la thymidine kinase.

### ANNEXE 1 : INDEX BIBLIOGRAPHIQUE

1. A.E. BUCKMASTER, S.D. SCOTT, M.J. SANDERSEN, M.E.G. BOURSNELL, L.J.N. ROSS, M.H. BINNS (1988) J. Gen. Virol., 69, 2033-2042
2. P.L. FELGNER, T.R. GADEK, M. HOLM, R. ROMAN, H.W. CHAN, M. WENZ, J.P. NORTHROP, G.M. RINGOLD and M. DANIELSEN (1987) Proc. Natl. Acad. Sci., USA, 84, 7413
3. H. FIELD, P. WILDY (1987) J. Hygiene (Cambridge), 81, 267-277
4. D.J. GOLDSTEIN, S.K. WELLER (1988) J. Virol., 62, 196-205
5. D.J. GOLDSTEIN, S.K. WELLER (1988) Virology, 166, 41-51
6. J. GOUGH et N. MURRAY (1983) J. Mol. Biol., 166, 1-19
7. J.G. JACOBSON, D.A. LEIB, D.J. GOLDSTEIN, C.L. BOGART, P.A. SCHAFFER, S.K. WELLER, D.M. COEN (1989) Virology, 173 (1), 276-283
8. A.T. JAMIESON et all. (1974) J. Gen. Virol., 24, 465-480
9. EP-A-O 316 658
10. T. KUNKEL (1985) Proc. Natl. Acad. Sci., 82, 488-492
11. T. KUNKEL, J. ROBERTS, B. ZAKOUR (1987) In Methods in Enzymology, 154, 367-382 Acad. Press
12. Y. LANGELIER, G. BUTTIN (1981) J. Gen. Virol., 57, 21-31
13. H. LANKINEN, A. GAASLUND, L. THELANDER (1982) J. Virol., 41, 893-900
14. LEE et al. (1980) J. Gen. Virol., 51, 245-253
15. J. McLAUCHLAN, J.B. CLEMENTS (1983) The Embo Journal, 2, (11), 1953-1961
16. J. McLAUCHLAN, J.B. CLEMENTS (1983) J. Gen. Virol., 64, 997-1006
17. T. MANIATIS, E.F. FRITSCH, J. SAMBROOK (1982) "Molecular cloning: A laboratory manual" Cold Spring Harbor Laboratory, Cold Spring Harbor, N.Y.
18. C.T. METTENLEITER, C. SCHREURS, F. ZUCKERMANN, T. BEN PORAT (1987) J. Virol., 61, 4030-4032
19. K. NAKAMAYE and F. ECKSTEIN (1985) Nucleic Acid Res., 13, 9679-9698
20. V.G. PRESTON, A.J. DARLING, I.M. McDOUGALL (1988) Virology, 167, 458-467
21. B. ROIZMAN et al. (1983) Cold Spring Harbor Conference on New Approaches to Viral Vaccines, September 1983
22. M. RUSSEL, S. KIDD, M. KELLEY (1986) Gene, 45, 333-338
23. P.J; SANDERS (1982) J. Gen. Virol., 63, 277-295
24. F. SANGER, S. NICKLEN, A.R. COULSON (1977) Proc. Natl. Acad., USA, 74, 5463-5467
25. S.K. SHAPIRA et al. (1983) Gene, 25, 71-82
26. M.F. SHIH, M. ARSENAKIS, P. THIOLLAIS, B. ROIZMAN (1984) Proc. Natl. Acad. Sci., USA, 81, 5867-5870
27. S. SIMPSON, J. McLAUCHLAN, J.B. CLEMENTS (1989) 14th International Herpesvirus Workshop, Nyborg-strand, Denmark (1989)
28. M.A. SWAIN, D.A. GALLOWAY (1986) J. Virol., 57, (3), 802-808
29. S. TABOR and C.C. RICHARDSON (1987) Proc. Natl. Acad. Sci., USA, 84, N°14, 4767-4771
30. G. TATAROV (1968) Zentralbl. Vet. Med., 15B, 848-853
31. J.W. TAYLOR et al. (1985) Nucleic Acid Res., 13, 8749-8765
32. J.W. TAYLOR, J. OTT, F. ECKSTEIN (1985) Nucleic Acid Res., 13, 8764-8785
33. R.L. THOMPSON, E.K. WAGNER, J.G. STEVENS (1983) Virology, 131, 180-192
34. D.R. THOMSEN, C.C. MARCHIOLI, R.J; YANCEY (1987) J. Virol., 61, 229-232
35. P.C. WEBER (1987) Science, 236, 576-579
36. R.L. WITTER et al. (1970) Am. J. Vet. Res., 31, 525-538
37. BOLIVAR F. et al., Gene 1977, 2, 95-113 38. YANNISCH-PERRON C. et al., Gene 1985, 33, 103-119.

### ANNEXE 2 : LISTE DES SEQUENCES

### SEQ ID NO:1

Longueur de la séquence : 3 278 paires de bases
Type de molécule séquencée :
ADN génomique
Origine de la molécule :
Virus herpès de la dinde (HVT) souche FC 126
Source expérimentale :
Plasmide pHVT K1
Caractéristiques :
de 1 à 1 662 paires de bases : gène RR1
de 1 663 à 1 694 paires de bases : région non codante
de 1 695 à 2 759 paires de bases : gène RR2
de 2 760 à 3 278 paires de bases : région non codante

GAT CCA AGA ACT ACA ACC ACC CAA GAT ACT ATC AAA GTT ATA ACG 45
Asp Pro Arg Thr Thr Thr Thr Gln Asp Thr Ile Lys Val Ile Thr 15

AAT GAT GTT GTT CCC CAT CTC CTG GCA CGA GGA GGG ATA GGC ATA 90
Asn Asp Val Val Pro His Leu Leu Ala Arg Gly Gly Ile Gly Ile 30

TCA TTG CAG CAC GTC AAT CAA AAA TCG GGT CTG ATG CAT GTT TTA 135
Ser Leu Gln His Val Asn Gln Lys Ser Gly Leu Met His Val Leu 45

AAG CTG ATA GAT TCA TTG ATT GTG GCC ACT AAT GTG AAC GAG TCC 180
Lys Leu Ile Asp Ser Leu Ile Val Ala Thr Asn Val Asn Glu Ser 60

CGG CCG ACG GGC GTT TGT GTG TAT TTG GAA CCC TGG CAT TCA GAC 225
Arg Pro Thr Gly Val Cys Val Tyr Leu Glu Pro Trp His Ser Asp 75

ATC ATG TCG GCT TTG ACG ATG CGC GGA ATG ATG GCC GCC GAG GAA 270
Ile Met Ser Ala Leu Thr Met Arg Gly Met Met Ala Ala Glu Glu 90

TCG AGA AGA TGT GAT AAT GTA TTC CTA GCT CTT TGG GCG TGC GAC 315
Ser Arg Arg Cys Asp Asn Val Phe Leu Ala Leu Trp Ala Cys Asp 105

CTC CTG TTT AAG AGA TAC CTG CGA TAT GTT AAT GGA GAA AAA AAT 360
Leu Leu Phe Lys Arg Tyr Leu Arg Tyr Val Asn Gly Glu Lys Asn 120

GTG ATG TGG ACT TTA TTT GAC TCC CGC GCG TCT ATT TTA TCA AAA 405
Val Met Trp Thr Leu Phe Asp Ser Arg Ala Ser Ile Leu Ser Lys 135

CTA TAT GGC GAT AAA TTC GAG GTG GAA TAT GAA CGT CTC GAA AAA 450
Leu Tyr Gly Asp Lys Phe Glu Val Glu Tyr Glu Arg Leu Glu Lys 150

GAA GGT ATT GGG GTG GCC CAA ATT CCA ATC AGA GAC ATG ATG TTT 495
Glu Gly Ile Gly Val Ala Gln Ile Pro Ile Arg Asp Met Met Phe 165

GCG ATC ATA AAA AGC GCA GCT TCT ACT GGA AGT CCA TTC ATT CTC 540
Ala Ile Ile Lys Ser Ala Ala Ser Thr Gly Ser Pro Phe Ile Leu 180

TTC AAA GAC GCC TGC AAC CGA CAT TAC ATC ACG GAC ACC CAG GGC 585
Phe Lys Asp Ala Cys Asn Arg His Tyr Ile Thr Asp Thr Gln Gly 195

GAT GCT ATT GCG GGA TCC AAT CTG TGC ACA GAA ATA ATA CAG AAA 630
Asp Ala Ile Ala Gly Ser Asn Leu Cys Thr Glu Ile Ile Gln Lys 210

ACA AAC GAA TCC ACA AAT GGC GTG TGC ACC CTA GCA AGC ATT AAT 675
Thr Asn Glu Ser Thr Asn Gly Val Cys Thr Leu Ala Ser Ile Asn 225

CTG GCC AGA TGC GTT CGC CGT GTT AAC GTG AAT GTA AAT TCG ATT 720
Leu Ala Arg Cys Val Arg Arg Val Asn Val Asn Val Asn Ser Ile 240

TTG ATG CCC TTA GGC ATG CCG TTT CGA CTG GCC ACC GTT TTT ACC 765
Leu Met Pro Leu Gly Met Pro Phe Arg Leu Ala Thr Val Phe Thr 255

AAT GCA ATA ATG GAT GGG AGT GAT GTC CCC ACA GTC AAA TCT CAA 810
Asn Ala Ile Met Asp Gly Ser Asp Val Pro Thr Val Lys Ser Gln 270

TCG GGT CGA GAC CGC AAC AGA TCT ATT GGT ATA GGC GTC CAA GGA 855
Ser Gly Arg Asp Arg Asn Arg Ser Ile Gly Ile Gly Val Gln Gly 285

TTT CAT ACA GCC ATG CTA TCT TTG GGT CTA GAT TTA GAG GAC GGA 900
Phe His Thr Ala Met Leu Ser Leu Gly Leu Asp Leu Glu Asp Gly 300

GCT GTC AGA GCA CTT AAT AAG CAA ATA TTT GAA CTA ATG CTA TTA 945
Ala Val Arg Ala Leu Asn Lys Gln Ile Phe Glu Leu Met Leu Leu 315

GAA GCT ATG ACC GTG AGC TGC GAA TTT TGT GAG CGG GGT CTT CCA 990

Glu Ala Met Thr Val Ser Cys Glu Phe Cys Glu Arg Gly Leu Pro 330

CCG TTC CCA GAC TTC TCT GAC AGC TAC TAT GCT CAA GGC CGT TTG 1035
Pro Phe Pro Asp Phe Ser Asp Ser Tyr Tyr Ala Gln Gly Arg Leu 345

CAT TTT GAT GGA TGG GAT AGT GTG GAG TTA ACG GCC CCC GAG GAA 1080
His Phe Asp Gly Trp Asp Ser Val Glu Leu Thr Ala Pro Glu Glu 360

TGG GGA GTT CTC CGC GGT CGT ATA ATG TCG TCT GGG CTT TAC AAC 1125
Trp Gly Val Leu Arg Gly Arg Ile Met Ser Ser Gly Leu Tyr Asn 375

GCC CAG TTC ATA GCG CTG ATG CCT ACT GCC GCA TCG GCG CAA GTG 1170
Ala Gln Phe Ile Ala Leu Met Pro Thr Ala Ala Ser Ala Gln Val 390

ACC GAG GTT AGC GAA GGA TTT GCC CCT TTG TTC AGT AAC ATG TTC 1215
Thr Glu Val Ser Glu Gly Phe Ala Pro Leu Phe Ser Asn Met Phe 405

AGC AAG GTG ACA AGT GCC GGG GAA CTG CTT AGA CCC AAC AGT CAA 1260
Ser Lys Val Thr Ser Ala Gly Glu Leu Leu Arg Pro Asn Ser Gln 420

TTA ATG CGG GAC GTG AGA CAG ATA TAT CCC GAT AAT GAG CAG CGT 1305
Leu Met Arg Asp Val Arg Gln Ile Tyr Pro Asp Asn Glu Gln Arg 435

CGC TTA AGC GCC ATT ACT GCA CTT GAG TCC ACT GCA TGG TGC GTC 1350
Arg Leu Ser Ala Ile Thr Ala Leu Glu Ser Thr Ala Trp Cys Val 450

AAA GAA GCG CTA GGG GAT CGG CCG GAA TGT ACT CGT CTA CTC AAA 1395
Lys Glu Ala Leu Gly Asp Arg Pro Glu Cys Thr Arg Leu Leu Lys 465

TAT AAA ACG GCG TTC GAA TAC GAT CAA TCT CTC CTA ATA GAT TTA 1440
Tyr Lys Thr Ala Phe Glu Tyr Asp Gln Ser Leu Leu Ile Asp Leu 480

TGT GCG GAT AGA GCC CCT TTT GTG GAT CAG AGC CAA TCA ATG ACT 1485
Cys Ala Asp Arg Ala Pro Phe Val Asp Gln Ser Gln Ser Met Thr 495

CTG TTT GTA ACG GAA ACA GCT GAC GGC ACG CTA TTG GCA TCC CGC 1530
Leu Phe Val Thr Glu Thr Ala Asp Gly Thr Leu Leu Ala Ser Arg 510

GTC ATG AAG CTC TTA CAT GCC TAT AAA AGC TGG TCT CAA AAC GGG 1575
Val Met Lys Leu Leu His Ala Tyr Lys Ser Trp Ser Gln Asn Gly 525

AAT GTA CTA TTG CAA GAT CGC AAG GCT ACG AAT ACT GGC ATA TTT 1620
Asn Val Leu Leu Gln Asp Arg Lys Ala Thr Asn Thr Gly Ile Phe 540

AGC GGC GAC GGA GAA CTG ACC TGT TCT TCC TGC GTG TTG TAATAAC 1666
Ser Gly Asp Gly Glu Leu Thr Cys Ser Ser Cys Val Leu 553

CACCGTTTATTAACTCAATATAGCCGCCATG AAC AAC CCA GTG CAT GCT GCC 1718
Met Asn Asn Pro Val His Ala Ala 561

GGG AAT CCG CCG AAC AAT TAT TTT TCT TTA GAT GGG ACC GAT CTT 1763
Gly Asn Pro Pro Asn Asn Tyr Phe Ser Leu Asp Gly Thr Asp Leu 576

CAT CTT TCC GAG AGA GGG GCC ACT TCT CCG AAA GGG AGT GAT GGG 1808
His Leu Ser Glu Arg Gly Ala Thr Ser Pro Lys Gly Ser Asp Gly 591

GGA GAC CTA GCC TCT CCG TAC GTG AAC AAT TGC CAT ATC ACA ACG 1853
Gly Asp Leu Ala Ser Pro Tyr Val Asn Asn Cys His Ile Thr Thr 606

GCG CAA TAT TTC TAC GTT CCG GAA TGC CCT GAT ATA GGA AAC CTA 1898
Ala Gln Tyr Phe Tyr Val Pro Glu Cys Pro Asp Ile Gly Asn Leu 621

CGA TCT TTG AGC ATC ATG AAC CGG TGG ACC GAA ACG GAA TTC GTA 1943
Arg Ser Leu Ser Ile Met Asn Arg Trp Thr Glu Thr Glu Phe Val 636

ATT GCA GAC GAC CTC GAG GAT GTC GGC AAG CTT AAG AAT GAA AAA 1988

Ile Ala Asp Asp Leu Glu Asp Val Gly Lys Leu Lys Asn Glu Lys 651

AAT TTT TAT CGC TTT CTA TTT ACC TTT TTA TCC GCC GCC GAC GAT 2033
Asn Phe Tyr Arg Phe Leu Phe Thr Phe Leu Ser Ala Ala Asp Asp 666

CTA GTT AAC TTG AAT ATA GAC AGT CTG TTG AGT TTA TTC ACT CAG 2078
Leu Val Asn Leu Asn Ile Asp Ser Leu Leu Ser Leu Phe Thr Gln 681

AAA GAT ATA CAT CAT TAT TAC TTT GAA CAG GAA TGT ATA GAA GCT 2123
Lys Asp Ile His His Tyr Tyr Phe Glu Gln Glu Cys Ile Glu Ala 696

GTC CAT TCG AGG GCC TAC AGT ATA ATT CAG CTA ATG CTG TTC AGC 2168
Val His Ser Arg Ala Tyr Ser Ile Ile Gln Leu Met Leu Phe Ser 711

AAT GAT CAA GCC GCT CGC CAA GAA TAC GTC ACC TCA ACT TTG AGA 2213
Asn Asp Gln Ala Ala Arg Gln Glu Tyr Val Thr Ser Thr Leu Arg 726

TCC CCC GCA ATT TTA TCA AAA TTG GAA TGG TTG GAA CGG CGA GTT 2258
Ser Pro Ala Ile Leu Ser Lys Leu Glu Trp Leu Glu Arg Arg Val 741

GCA GAA TGC ACC TCT ATC GCT GAA AAA TAT ATT CTC ATG ATT TTA 2303
Ala Glu Cys Thr Ser Ile Ala Glu Lys Tyr Ile Leu Met Ile Leu 756

ATA GAG GGT ATA TTT TTC ACT GCG TCT TTT GCT GCA ATC GCC TAC 2348
Ile Glu Gly Ile Phe Phe Thr Ala Ser Phe Ala Ala Ile Ala Tyr 771

CTT CGT GTC AAT AAC CTG TTT GTG GTT ACA TGT CAA ATT AAC AAC 2393
Leu Arg Val Asn Asn Leu Phe Val Val Thr Cys Gln Ile Asn Asn 786

TTG ATT AGC AGA GAT GAA GCT ATA CAC GTG GAA GCA TCC TGT TGC 2438
Leu Ile Ser Arg Asp Glu Ala Ile His Val Glu Ala Ser Cys Cys 801

ATT TTT AAA AAT TAT CTC GCC GGC CCC AAA CCT ACT ACT GCC CGC 2483
Ile Phe Lys Asn Tyr Leu Ala Gly Pro Lys Pro Thr Thr Ala Arg 816

ATC CAC ACG CTG TTT AAA GAA GCC GTT ACG GTG GAA TGC GAG TTC 2528
Ile His Thr Leu Phe Lys Glu Ala Val Thr Val Glu Cys Glu Phe 831

CTC CGC ACG GCG GCT CCT CGC ACC AGT AAT ATT ATC AAT ATT GAT 2573
Leu Arg Thr Ala Ala Pro Arg Thr Ser Asn Ile Ile Asn Ile Asp 846

GCC ATT TGC AGC TAT GTA CGG TAC AGT GCA GAC AGG TTG TTG AGA 2618
Ala Ile Cys Ser Tyr Val Arg Tyr Ser Ala Asp Arg Leu Leu Arg 861

GCG CTT GAT ATA CTG CCC ATT TAC GAC GAA CCC AAA CCC CCT GCT 2663
Ala Leu Asp Ile Leu Pro Ile Tyr Asp Glu Pro Lys Pro Pro Ala 876

GAT TTC CCC CTC GTC CTC ATG TCC GCT GCA AGC AAT ACT AAC TTT 2708
Asp Phe Pro Leu Val Leu Met Ser Ala Ala Ser Asn Thr Asn Phe 891

TTC GAG CGA CGA AAC ACC GCA TAC TCT GGA AGC GTT TCA AAT GAT 2753
Phe Glu Arg Arg Asn Thr Ala Tyr Ser Gly Ser Val Ser Asn Asp 906

CTT TAATTCGCAATTGAAATTACCCGATTCACGTGTACTTTAGGTCAAATATAAAGTT 2811
Leu 907

CGTGTAATGCATCCTCGTTCGCGTTTCTTTTTTAGGCGACCCTATTCCAATACTTTGTCA 2871

ACCACTCTATTGAAGGCGTATCTCGATGCGTCGTAAAAAGCGGATGCTAAACTGCCCGCT 2931

TCGTTACTATCAACTATACTACGGAGGACCAAGTTCTCAATTGCAGAATCATCCCGCGTC 2991

TCTTGCGTAATAGGAAACCGCTTTAAGATACTGACTCTGTGTGTTCTTCGTTCTGGTGTT 3051

ATATTTTCTATTACATGTTTTATAAATTTATATTCTAGAACTTCACATTTGGTTCGGTGG 3111

AGCTCGTAAATGCGTTAACGCCTGCCGTGCGTCGCGTATTATATTTACATCGTTATAGGT 3171
GGCGCACAGGCGGTCTGTGCTGGAGTTATGATCATTTTTGCGGTTCTCGCTTAAAGTTGT 3231
CCGTAGATTATGCTTCAGTCCAGACCTATCTATATGCTTCTCGTTTA 3278

### SEQ ID NO: 2

Longueur de la séquence : 2 176 paires de bases
Type de molécule séquencée : ADNc pour ARN génomique
Origine de la molécule :
Virus de la maladie de Newcastle
souche Texas
Source expérimentale :
Plasmide pNDV 108
Caractéristiques :
de 1 à 271 paires de bases : gène Matrix
de 272 à 430 paires de bases : région non codante
de 431 à 2 092 paires de bases : gène de la protéine fusion
de 2 093 à 2 176 paires de bases : région non codante

A CCT TCC GTG CTC GTG AAG GCG AGA GGT GCA CGG ACT AGG CTG 43
Pro Ser Val Leu Val Lys Ala Arg Gly Ala Arg Thr Arg Leu 14

CTG GCA CCT TTC TTC TCT AGC AGT GGG ACA GCC TGC TAT CCT ATA 88
Leu Ala Pro Phe Phe Ser Ser Ser Gly Thr Ala Cys Tyr Pro Ile 29

GCA AAT GCC TCT CCT CAG GTA GCT AAG ATA CTC TGG AGT CAA ACT 133
Ala Asn Ala Ser Pro Gln Val Ala Lys Ile Leu Trp Ser Gln Thr 44

GCG CGC CTG CGG AGT GTA AAA ATC ATC ATT CAA GCG GGC ACC CAA 178
Ala Arg Leu Arg Ser Val Lys Ile Ile Ile Gln Ala Gly Thr Gln 59

CGC GCT GTC GCA GTG ACT GCT GAC CAT GAG GTT ACC TCT ACT AAG 223
Arg Ala Val Ala Val Thr Ala Asp His Glu Val Thr Ser Thr Lys 74

ATA GAG AAG AGG CAT ACC ATT GCT AAA TAC AAT CCT TTC AAG AAA 268
Ile Glu Lys Arg His Thr Ile Ala Lys Tyr Asn Pro Phe Lys Lys 89

TAGGCTGCATCTCTGAGACTGCAATCCGCCCGCTTTCCCGAATCACCATGATACTAGATA 328

ATGATCTGTCTTGATTGCTTACAGTTAGTTTACCTGTCTATCAAGTTAGAAAAACACGG 388

GTAGAAGAATTTGGATCCCGGTTGGCACATTCAAGGTGCAAGATG GGC TCC AGA 442
Met Gly Ser Arg 93

TCT TCT ACC AGG ATC CCG GTA CCT CTA ATG CTG ATC ATC CGA ACC 487
Ser Ser Thr Arg Ile Pro Val Pro Leu Met Leu Ile Ile Arg Thr 108

GCG CTG ACA CTG AGC TGT ATC CGT CTG ACA AGC TCT CTT GAT GGC 532
Ala Leu Thr Leu Ser Cys Ile Arg Leu Thr Ser Ser Leu Asp Gly 123

AGG CCT CTT GCG GCT GCA GGG ATC GTG GTA ACA GGA GAT AAA GCA 577
Arg Pro Leu Ala Ala Ala Gly Ile Val Val Thr Gly Asp Lys Ala 138

GTC AAC ATA TAC ACC TCA TCC CAG ACA GGG TCA ATC ATA GTT AAG 622
Val Asn Ile Tyr Thr Ser Ser Gln Thr Gly Ser Ile Ile Val Lys 153

TTA CTC CCG AAT ATG CCC AAG GAC AAA GAG GTG TGT GCA AAA GCC 667
Leu Leu Pro Asn Met Pro Lys Asp Lys Glu Val Cys Ala Lys Ala 168

CCA TTG GAG GCA TAC AAC AGG ACA CTG ACT ACT TTA CTC ACC CCC 712
Pro Leu Glu Ala Tyr Asn Arg Thr Leu Thr Thr Leu Leu Thr Pro 183

CTT GGT GAT TCT ATC CGC AGG ATA CAA GAG TCT GTG ACT ACT TCC 757
Leu Gly Asp Ser Ile Arg Arg Ile Gln Glu Ser Val Thr Thr Ser 198

G[?][?] GGA AGG AGA CAG AGA CGC TTT ATA GGT GCC ATT ATC GGC AGT 802
Gly Gly Arg Arg Gln Arg Arg Phe Ile Gly Ala Ile Ile Gly Ser 213

GTA GCT CTT GGG GTT GCG ACA GCT GCA CAG ATA ACA GCA GCT TCG 847
Val Ala Leu Gly Val Ala Thr Ala Ala Gln Ile Thr Ala Ala Ser 228

GCC CTG ATA CAA GCC AAC CAG AAT GCT GCC AAC ATC CTC CGG CTT 892
Ala Leu Ile Gln Ala Asn Gln Asn Ala Ala Asn Ile Leu Arg Leu 243

AAA GAG AGC ATT GCT GCA ACC AAT GAA GCT GTG CAC GAG GTC ACT 937
Lys Glu Ser Ile Ala Ala Thr Asn Glu Ala Val His Glu Val Thr 258

GAC GGA TTA TCA CAA CTA GCA GTG GCA GTA GGG AAG ATG CAA CAG 982
Asp Gly Leu Ser Gln Leu Ala Val Ala Val Gly Lys Met Gln Gln 273

TTT GTC AAT GAC CAG TTC AAT AAT ACA GCG CAA GAA TTG GAC TGT 1027
Phe Val Asn Asp Gln Phe Asn Asn Thr Ala Gln Glu Leu Asp Cys 288

ATA AAA ATT GCA CAG CAG GTC GGT GTA GAA CTC AAC TTG TAC CTA 1072
Ile Lys Ile Ala Gln Gln Val Gly Val Glu Leu Asn Leu Tyr Leu 303

ACT GAA TTG ACT ACA GTA TTT GGG CCA CAA ATC ACT TCC CCT GCC 1117
Thr Glu Leu Thr Thr Val Phe Gly Pro Gln Ile Thr Ser Pro Ala 318

TTA ACT CAG CTG ACT ATC CAA GCG CTT TAC AAT CTA GCT GGT GGT 1162
Leu Thr Gln Leu Thr Ile Gln Ala Leu Tyr Asn Leu Ala Gly Gly 333

AAT ATG GAT TAC TTG CTG ACT AAG TTA GGT GTA GGG AAC AAC CAA 1207
Asn Met Asp Tyr Leu Leu Thr Lys Leu Gly Val Gly Asn Asn Gln 348

CTC AGC TCA TTA ATT GGT AGC GGC TTG ATC ACC GGC AAC CCT ATT 1252
Leu Ser Ser Leu Ile Gly Ser Gly Leu Ile Thr Gly Asn Pro Ile 363

CTG TAC GAC TCA CAG ACT CAG ATC TTG GGT ATA CAG GTA ACT TTG 1297
Leu Tyr Asp Ser Gln Thr Gln Ile Leu Gly Ile Gln Val Thr Leu 378

CCT TCA GTT GGG AAC CTG AAT AAT ATG CGT GCC ACC TAC CTG GAG 1342
Pro Ser Val Gly Asn Leu Asn Asn Met Arg Ala Thr Tyr Leu Glu 393

ACC TTA TCT GTA AGC ACA ACC AAG GGA TTT GCC TCA GCA CTT GTC 1387
Thr Leu Ser Val Ser Thr Thr Lys Gly Phe Ala Ser Ala Leu Val 408

CCA AAA GTG GTG ACA CAG GTC GGT TCC GTG ATA GAA GAA CTT GAC 1432
Pro Lys Val Val Thr Gln Val Gly Ser Val Ile Glu Glu Leu Asp 423

ACC TCA TAC TGT ATA GGG ACC GAC TTG GAT TTA TAC TGT ACA AGA 1477
Thr Ser Tyr Cys Ile Gly Thr Asp Leu Asp Leu Tyr Cys Thr Arg 438

ATA GTG ACA TTC CCT ATG TCT CCT GGT ATT TAT TCT TGT CTG AGC 1522
Ile Val Thr Phe Pro Met Ser Pro Gly Ile Tyr Ser Cys Leu Ser 453

GGT AAT ACA TCG GCT TGC ATG TAT TCA AAG ACT GAA GGC GCA CTT 1567
Gly Asn Thr Ser Ala Cys Met Tyr Ser Lys Thr Glu Gly Ala Leu 468

ACT ACG CCA TAT ATG GCT CTC AAA GGC TCA GTT ATT GCC AAT TGC 1612
Thr Thr Pro Tyr Met Ala Leu Lys Gly Ser Val Ile Ala Asn Cys 483

AAG CTG ACA ACA TGT AGA TGT GCA GAT CCC CCA GGT ATC ATA TCG 1657
Lys Leu Thr Thr Cys Arg Cys Ala Asp Pro Pro Gly Ile Ile Ser 498

CAA AAT TAT GGA GAA GCT GTG TCC TTA ATA GAT AGG CAC TCA TGC 1702
Gln Asn Tyr Gly Glu Ala Val Ser Leu Ile Asp Arg His Ser Cys 513

AAC GTC TTA TCC TTA GAC GGG ATA ACT CTG AGG CTC AGT GGG GAA 1747
Asn Val Leu Ser Leu Asp Gly Ile Thr Leu Arg Leu Ser Gly Glu 528

TTT GAT GCA ACC TAT CAA AAG AAT ATC TCT ATA CTA GAT TCT CAA 1792
Phe Asp Ala Thr Tyr Gln Lys Asn Ile Ser Ile Leu Asp Ser Gln 543

GTT ATA GTG ACA GGC AAT CTT GAT ATA TCA ACT GAG CTT GGG AAT 1837
Val Ile Val Thr Gly Asn Leu Asp Ile Ser Thr Glu Leu Gly Asn 558

GTC AAC AAC TCA ATA AGT AAT GCC CTG AAT AAG TTA GAG GAA AGC 1882
Val Asn Asn Ser Ile Ser Asn Ala Leu Asn Lys Leu Glu Glu Ser 573

AAC AGC AAA CTA GAC AAA GTC AAT GTC AAA CTG ACC AGC ACA TCT 1927
Asn Ser Lys Leu Asp Lys Val Asn Val Lys Leu Thr Ser Thr Ser 588

GCT CTC ATT ACC TAC ATC GTT TTA ACT GTC ATA TCT CTT GTT TTT 1972
Ala Leu Ile Thr Tyr Ile Val Leu Thr Val Ile Ser Leu Val Phe 603

GGT GTA CTT AGC CTG GTT CTA GCA TGC TAC CTG ATG TAC AAG CAA 2017
Gly Val Leu Ser Leu Val Leu Ala Cys Tyr Leu Met Tyr Lys Gln 618

AAG GCA CAA CAA AAG ACC TTG TTA TGG CTT GGG AAT AAT ACC CTT 2062
Lys Ala Gln Gln Lys Thr Leu Leu Trp Leu Gly Asn Asn Thr Leu 633

GAT CAG ATG AGA GCC ACT ACA AAA ATA TGAATACAAACGAGAGGCGGAGG 2112
Asp Gln Met Arg Ala Thr Thr Lys Ile 642

TATCCCCAATAGCAATTTGCGTGTAAATTCTGGCAACCTGTTAATTAGAAGAATTAAGAA 2172

AAAA

### SEQ ID NO : 3

TYPE DE SEQUENCE : oligonucléotide
LONGUEUR DE LA SEQUENCE : 21 bases
TYPE DE MOLECULE : ADN

5' GCAGCATGCCCGGGGTTGTTG 3'
SmaI

### SEQ ID NO : 4

TYPE DE SEQUENCE : oligonucléotide
LONGUEUR DE LA SEQUENCE : 21 bases
TYPE DE MOLECULE : ADN

5' CCGATTCACGTCGACTTTAGG 3'
SalI

### SEQ ID NO : 5

TYPE DE SEQUENCE : oligonucléotide
LONGUEUR DE LA SEQUENCE : 21 bases
TYPE DE MOLECULE : ADN

5' GGGTTGTCCATGGCGGCTATA 3'
NcoI

### SEQ ID NO : 6

TYPE DE SEQUENCE : oligonucléotide
LONGUEUR DE LA SEQUENCE : 22 bases
TYPE DE MOLECULE : ADN

5' CAAGGTGCACCATGGGCTCCAG 3'
NcoI

### SEQ ID NO : 7

TYPE DE SEQUENCE : oligonucléotide
LONGUEUR DE LA SEQUENCE : 25 bases
TYPE DE MOLECULE : ADN

5' GCTATTGGGGATATCTCCGCCTCTC 3'
EcoRV

## Revendications

1. Virus recombinant HVT, comprenant au moins un gène hétérologue inséré dans la région, du génome dudit virus, correspondant au gène de la petite sous-unité RR2 de la ribonucléotide réductase, de façon à pouvoir être exprimé.

2. Virus recombinant selon la revendication 1, caractérisé en ce que le gène hétérologue code pour un immunogène viral, bactérien ou parasitaire.

3. Virus recombinant HVT, comprenant au moins un gène hétérologue qui est inséré dans la région, du génome de HVT, correspondant au gène de la petite sous-unité RR2 de la ribonucléotide réductase et qui code pour des immunogènes provenant d'agents pathogènes associés notamment à la maladie de Marek, à la bronchite infectieuse aviaire, à la maladie de Newcastle, à la peste aviaire, à la maladie de Gumboro, à la laryngotrachéite infectieuse, à la coccidiose, à la colibaccillose.

4. Virus recombinant selon la revendication 2 ou 3, caractérisé en ce que le gène hétérologue inséré est susceptible d'être exprimé sous le contrôle des séquences régulatrices de la transcription du gène de la petite sous-unité RR2.

5. Virus recombinant selon la revendication 2 ou 3, caractérisé en ce que le gène hétérologue inséré est susceptible d'être exprimé sous la dépendance de séquences promotrices, du virus considéré ou d'autres virus herpès, rapportées dans le génome du virus considéré.

6. Virus recombinant selon l'une des revendications 2 à 5, caractérisé en ce que les codons d'initiation et de terminaison du gène RR2 sont remplacés par ceux du gène à insérer.

7. Procédé de préparation d'un virus recombinant à partir d'un virus HVT, caractérisé en ce qu'on insère au moins un gène hétérologue dans la région, du génome dudit virus, qui correspond au gène de la petite sous-unité RR2 de la ribonucléotide réductase, de telle façon que ledit gène hétérologue puisse être exprimé.

8. Procédé selon la revendication 7, caractérisé en ce qu'on isole une partie du génome viral renfermant le gène de la petite sous-unité RR2, on réalise une délétion partielle ou totale de ce gène et on insère le gène hétérologue dans la région correspondant audit gène avant d'insérer la séquence ADN obtenue dans le génome du virus par cotransfection et recombinaison homologue.

9. Procédé selon la revendication 7 ou 8, caractérisé en ce que le gène inséré est sous le contrôle de séquences régulatrices de la transcription du gène RR2.

10. Procédé selon la revendication 7 ou 8, caractérisé en ce que le gène inséré est sous la dépendance de séquences promotrices, du virus considéré ou d'autres virus herpès, rapportées dans le génome du virus considéré.

11. Procédé selon l'une des revendications 7 à 10, caractérisé en ce que l'on substitue les codons d'initiation et de terminaison du gêne RR2 par ceux du gène à insérer.

12. Procédé de préparation d'un virus recombinant HVT, caractérisé en ce que :
a) on isole un fragment Bam HI K1 du génome de HVT par digestion du génome par l'enzyme de restriction Bam HI
b) on digère ce fragment par l'enzyme de restriction Hind III, pour obtenir un fragment correspondant à la partie 5' du gène RR2 et à la région en amont incluant le promoteur et un fragment correspondant à la partie 3' du gène RR2 et à la région en aval de ce gène,
c) on clone les deux fragments 5' et 3' obtenus en b) respectivement dans les vecteurs pUC18 et pUC19,
d) on digère ces plasmides respectivement par les couples d'enzymes de restriction Hind III - Aat II et Xmn I - Aat II puis on les ligue ensemble pour donner un nouveau plasmide comportant une délétion entre les sites initiaux Hind III et Xmn I,
e) on crée par mutagénèse dirigée les sites de restriction dans le plasmide obtenu en d),
f) on clone dans ce site de restriction un gène hétérologue, et
g) on insère le fragment d'ADN obtenu en f) dans le génome du virus HVT par cotransfection et recombinaison homologue.

13. Procédé selon la revendication 12, caractérisé en ce que le gène hétérologue code pour un immunogène provenant d'agents pathogènes associés à une maladie aviaire telle que la maladie de Marek, la bronchite infectieuse aviaire, la maladie de Newcastle, la peste aviaire, la maladie de Gumboro, la laryngotrachéite infectieuse, la coccidiose, la colibacillose.

14. Virus recombinant obtenu selon l'une quelconque des revendications 7 à 13.

15. Vaccin comprenant un virus recombinant selon l'une quelconque des revendications 1 à 6 et 14.

16. Séquence nucléotidique délimitée par les bases 1695 à 2759 à la SEQ ID NO : 1, et ses variantes et fragments, et correspondant au gène de la petite sous-unité RR2 de la ribonucléotide réductase du virus HVT.

## Patentansprüche

1. Rekombinantes HVT-Virus, enthaltend mindestens ein heterologes Gen, das in den Bereich des Genoms des genannten Virus, der dem Gen der kleinen Untereinheit RR2 der Ribonucleotid-Reduktase entspricht, in der Weise eingefügt ist, daß es exprimiert werden kann.

2. Rekombinantes Virus nach Anspruch 1, **dadurch gekennzeichnet,** daß das heterologe Gen für ein virales, bakterielles oder parasitäres Immunogen codiert.

3. Rekombinantes HVT-Virus, enthaltend mindestens ein heterologes Gen, welches in den Bereich des HVT-Genoms, der dem Gen der kleinen Untereinheit RR2 der Rebonucleotid-Reduktase entspricht, eingefügt ist und für Immunogene codiert, die von pathogenen Mitteln abstammen, welche insbesondere mit der Marek-Krankheit, der infektiösen Vogel-Bronchitis, der Newcastle-Krankheit, der Vogel-Pest, der Gumboro-Krankheit, der infektiösen Laryngotracheitis, der Kokzidiose und der Colibazillose verknüpft ist.

4. Rekombinantes Virus nach Anspruch 2 oder 3, **dadurch gekennzeichnet,** daß das eingefügte heterologe Gen unter der Steuerung von regulierenden Sequenzen der Transkription des Gens der kleinen Untereinheit RR2 exprimiert werden kann.

5. Rekombinantes Virus nach Anspruch 2 oder 3, **dadurch gekennzeichnet,** daß das eingefügte heterologe Gen in Abhängigkeit von fördernden Sequenzen des in Rede stehenden Virus oder anderer Herpesviren, die in dem Genom des in Rede stehenden Virus eingebracht sind, exprimiert weden kann.

6. Rekombinantes Virus nach einem der Ansprüche 2 bis 5, **dadurch gekennzeichnet,** daß die Start- und Stop-Codons des Gens RR2 durch jene des einzufügenden Gens ersetzt sind.

7. Verfahren zur Herstellung eines rekombinanten Virus ausgehend von einem HVT-Virus, **dadurch gekennzeichnet,** daß man mindestens ein heterologes Gen in den Beireich des Genoms des genannten Virus, der dem Gen der kleinen Untereinheit RR2 der Ribonucleotid-Reduktase entspricht, in der Weise einfügt, daß das heterologe Gen exprimiert werden kann.

8. Verfahren nach Anspruch 7, **dadurch gekennzeichnet,** daß man einen Teil des viralen Genoms, der das Gen der kleinen Untereinheit RR2 enthält, isoliert, eine teilweise oder vollständige Deletion dieses Gens bewirkt und das heterologe Gen in den dem genannten Gen entsprechenden Bereich einfügt, bevor man die erhaltenen DNA-Sequenz durch Co-Transfektion und homologe Rekombination in das Genom des Virus einfügt.

9. Verfahren nach Anspruch 7 oder 8, **dadurch gekennzeichnet,** das das eingefügte Gen unter der Steuerung von regulierenden Sequenzen der Transkription des Gens RR2 steht.

10. Verfahren nach Anspruch 7 oder 8, **dadurch gekennzeichnet,** daß das eingefügte Gen unter Abhängigkeit von fördernden Sequenzen des in Rede stehenden Virus oder anderer Herpes-Viren, die in dem Genom des in Rede stehenden Virus vorliegen, steht.

11. Verfahren nach einem der Ansprüche 7 bis 10, **dadurch gekennzeichnet,** daß man die Start- und Stop-Codons des Gens RR2 durch jene des einzufügenden Gens ersetzt.

12. Verfahren zur Herstellung eines rekombinanten HVT-Virus, **dadurch gekennzeichnet,** daß man:
a) ein Fragment Bam HI K1 des HVT-Genoms durch Digerieren des Genoms mit dem Restriktionsenzym Bam HI isoliert,
b) dieses Fragment mit dem Restriktionsenzym Hind III digeriert, um ein Fragment, das dem 5'-Abschnitt des Gens RR2 und dem oberhalb davon liegenden Bereich einschließlich des Promotors entspricht, und ein Fragment, das dem 3'-Abschnitt des Gens RR2 und dem unterhalb davon liegenden Bereich dieses Gens entspricht, zu erhalten,
c) die beiden in der Stufe b) erhaltenen Fragmente 5' und 3' in den Vektoren pUC18 bzw. pUC19 kloniert.
d) diese Plasmide mit den Restriktionsenzympaaren Hind III - Aat II bzw. Xmn I - Aat II digeriert und sie anschließend verknüpft zur Bildung eines neuen Plasmids, welches eine Deletion zwischen den Ursprungsstellen Hind III und Xmn I aufweist,
e) durch gesteuerte Mutagenese Restriktionsstellen in dem in der Stufe d) erhaltenen Plasmid erzeugt,
f) in dieser Restriktionsstelle ein heterologes Gen kloniert und
g) das in der Stufe f) erhaltene DNA-Fragment durch Co-Transfektion und homologe Rekombination in das Genom des HVT-Virus einfügt.

13. Verfahren nach Anspruch 12, **dadurch gekennzeichnet,** daß das heterologe Gen für ein Immunogen codiert, das von pathogenen Mitteln stammt, die mit einer Vogel-Krankheit, wie der Marek-Krankheit, der infektiösen Vogelbronchitis, der Newcastle-Krankheit, der Vogel-Pest, der Gumboro-Krankheit, der infektiösen Laryngotracheitis, der Kokzidiose und der Colibazillose, verknüpft sind.

14. Rekombinantes Virus, erhalten nach einem der Ansprüche 7 bis 13.

15. Impfstoff, enthaltend ein rekombinantes Virus nach einem der Ansprüche 1 bis 6 und 14.

16. Nucleotidsequenz, begrenzt durch die Basen 1695 bis 2759 der SEQ ID No: 1 und dessen Varianten und Fragmente, entsprechend dem Gen der kleinen Untereinheit RR2 der Ribonucleotid-Reduktase des HVT-Virus.

## Claims

1. Recombinant HVT virus, comprising at least one heterologous gene inserted into the region of the genome of the said virus corresponding to the gene of the small sub-unit RR2 of the reductase ribonucleotide, in such a way as to be capable of being expressed.

2. Recombinant virus as claimed in Claim 1, characterised in that the heterologous gene codes for a viral, bacterial or parasitic immunogen.

3. Recombinant HVT virus, comprising at least one heterologous gene which is inserted in to the region of the HVT genome corresponding to the gene of the small sub-unit RR2 of the reductase ribonucleotide and which codes for immunogens originating from pathogenic agents associated particularly with Marek's disease, infectious bronchitis in fowl, Newcastle disease, fowlpest, Gumboro disease, infectious laryngotracheitis, coccidiosis, colibacillosis.

4. Recombinant virus as claimed in Claim 2 or 3, characterised in that the inserted heterologous gene is capable of being expressed under the control of the regulatory sequences of the transcription of the gene of the small sub-unit RR2.

5. Recombinant virus as claimed in Claim 2 or 3, characterised in that the inserted heterologous gene is capable of being expressed dependent upon promoting sequences of the virus concerned or of other herpes viruses put in the genome of the virus concerned.

6. Recombinant virus as claimed in one of Claims 2 to 5, characterised in that the codons for initiation and termination of the gene RR2 are replaced by those of the gene to be inserted.

7. Method of preparing a recombinant virus from a HVT virus, charaterised in that at least one heterologous gene is inserted in the region of the genome of the said virus which corresponds to the gene of the small sub-unit RR2 of the reductase ribonucleotide, in such a way that the said heterologous gene can be expressed.

8. Method as claimed in Claim 7, characterised in that a part of the viral genome including the small sub-unit RR2 is isolated, a partial or total deletion of this gene in carried out and the heterologous gene is inserted in the region corresponding to the said gene before the DNA sequence obtained is inserted in the genome of the virus by cotransfection and homologous recombination.

9. Method as claimed in Claim 7 or 8, characterised in that the inserted gene is under the control of regulatory sequences of the transcription of the gene RR2.

10. Method as claimed in Claim 7 or 8, characterised in that the inserted gene is dependent upon promoting sequences of the virus concerned or of other herpes viruses put in the genome of the virus concerned.

11. Method as claimed in one of Claims 7 to 10, characterised in that the codons for initiation and termination of the gene RR2 are substituted by those of the gene to be inserted.

12. Method of preparing a recombinant HVT virus, characterised in that:
a) a fragment Bam HI K1 of the HVT genome is isolated by digestion of the genome by the restricting enzyme Bam HI.
b) this fragment is digested by the restricting enzyme Hind III in order to obtain a fragment corresponding to the part 5' of the gene RR2 and to the region upstream including the promoter and a fragment corresponding to the part 3' of the gene RR2 and to the region downstream of this gene,
c) the two fragments 5' and 3' obtained in b) are cloned respectively in the vectors pUC18 and pUC19.
d) these plasmides are digested respectively by the pairs of restricting enzymes Hind III - Aat II and Xmn I - Aat II, then they are bonded together to give a new plasmide having a deletion between the initial sites Hind III and Xmn I,
e) restriction sites are created by directed mutagenesis in the plasmide obtained in d),
f) a heterologous gene is cloned in this restriction site, and
g) the DNA fragment obtained in f) is inserted in the genome of the HVT virus by cotransfection and homologous recombination.

13. Method as claimed in Claim 12, characterised in that the heterologous gene codes for an immunogen originating from pathogenic agents associated with a disease of fowl such as Marek's disease, infectious bronchitis in fowl, Newcastle disease, fowlpest, Gumboro disease, infectious laryngotracheitis, coccidiosis, colibacillosis.

14. Recombinant virus obtained according to any one of Claims 7 to 13.

15. Vaccine comprising a recombinant virus according to any one of Claims 1 to 6 and 14.

16. Nucleotide sequence delimited by the bases 1695 to 2759 in SEQ ID NO: 1, and variants and fragments thereof, and corresponding to the gene of the small sub-unit RR2 of the reductase ribonucleotide of the HVT virus.
